# EUROPEAN PATENT APPLICATION

(11) **EP 3 146 915 A1**
(43) Date of publication of application: **29.03.2017**
(21) Application number: 14885134.8
(22) Date of filing: 31.12.2014
(51) Int. Cl.: A61B 17/12

(54) **LEFT ATRIAL APPENDAGE OCCLUDER AND PREPARATION METHOD THEREOF**

(30) Priority: 10.03.2014 CN 201410085888
(71) Applicant: Shanghai Shape Memory Alloy Co., Ltd., Shanghai 201612 (CN)
(72) Inventor: WU, Hong, Shanghai 201612 (CN); ZHOU, Erchen, Shanghai 201612 (CN); ZHU, Yufeng, Shanghai 201612 (CN); YANG, Yongsen, Shanghai 201612 (CN); QIN, Yongwen, Shanghai 201612 (CN); CHEN, Juan, Shanghai 201612 (CN); CHU, Guojun, Shanghai 201612 (CN)
(74) Representative: Herrero & Asociados, S.L.
(86) International application number: PCT/CN2014/095889
(87) International publication number: WO 2015/135369

(57) **Abstract**

The present invention provides a left atrial appendage occluder and a manufacture method thereof, wherein the left atrial appendage occluder comprises a nitinol meshed bracket having a tubular shape and being provided with a left disk and a right disk at two ends of the nitinol meshed bracket, respectively; the left disk and the right disk are connected by a mobile portion, the left disk and the right disk are provided with a resistance membrane, respectively. The manufacture method of the left atrial appendage occluder comprises steps of weaving a elongated web, then performing pre-heat treatment, shaping, and final-heat treatment, etc., to accomplish the manufacture of the left atrial appendage occluder. The present invention is simple in structure, convenient in use, and can simplify the operation process, and shorten the operation time. Meanwhile, the present invention is no need for X-ray to perform the left atrial appendage occlusion surgery, which greatly reduces the damage to both the doctor and patient. Moreover, the left atrial appendage occluder manufactured by the manufacture method of the present invention has good performance, and is reliable in operation.

## Description

### Background of the Present Invention

### Field of Invention

The present invention relates to a medical instrument of a cardiac operation, and particularly relates to a left atrial appendage occluder and a manufacture method thereof.

### Description of Related Arts

Atrial fibrillation is one of the most common cardiac arrhythmias in clinical diseases, with a population incidence of about 0.89%. Wherein, the incidence increases with age, and may be up to 10% within the population above 75-year-old. Atrial fibrillation may result in an atrial activation frequency of 300∼600 beats per minute, as well as generally quick and irregular heartbeat frequency, while occasionally the atrial activation frequency may be up to 100∼160 beats per minute, which is not only much quicker than normal heartbeat, but also is irregular, and thus causes a lose of an effective systolic function of the atrium. The increase of the prevalence of the atrial fibrillation is also closely related to diseases, e.g., the coronary heart disease, hypertension and heart failure, etc. Generally, treatment for atrial fibrillation has been mainly centered on drug therapy, which enables to recover and maintain sinus rhythms, control ventricular rates and prevent thromboembolic events. With the development of medical standard, a variety of non-drug therapy has been studied, e.g., electrical conversion, catheter ablation, surgical maze surgery and anticoagulation therapy.

Besides inducing and aggravating the heart failure, the atrial fibrillation may also cause cerebral apoplexy and other thromboembolic events, wherein about 5% of the patients with atrial fibrillation occur the cerebral apoplexy every year, and the fatality rate and disability rate caused by the cerebral apoplexy is up to 50%, therefore, it is of great significance on how to prevent or reduce the cerebral apoplexy caused by the atrial fibrillation thromboembolism. In recent years, many clinical studies have shown that the risk of the cerebral apoplexy of the patients with atrial fibrillation can be reduced by occluding the left atrial appendage. Currently, the mainstream left atrial appendage occlusion in the world is percutaneous occlusion, which has complicated operation process and requires for puncturing atrial septum, thereby the operation is relative difficult, and the apparatus thereof is relative complicated. Moreover, during the operation process, an x-ray is generally used as a guide, which has radiation damage on both doctor and the patient.

A Chinese patent, with a patent number of CN 203263451 U, has disclosed a percutaneous left atrial appendage occluder, which comprises: a hemisphere end steel sleeve, a hemisphere, a hemisphere end polyester membrane, a hexagonal disk surface polyester membrance, a hexagonal disk, a cone end polyester membrance, a cone and a core end steel sleeve; wherein the hemisphere and the cone form a meshed bracket woven by super-elastic memory alloy wires, a hexagonal disk is arranged between the hemisphere and the cone, polyester membranes are arranged in the hexagonal disk; a nickel-titanium wire of the hemisphere is stringed to the center of the hemisphere and is fixed on a hemisphere steel sleeve; the hemisphere steel sleeve is provided with a precision internal thread, and can be screwed in and screwed out with a bolt at the far end of a push steel cable; a nickel-titanium alloy wire of the cone body is stringed to the center of the bottom of the cone and is fixedly provided with a cone end steel sleeve, the stringed part of the cone end steel sleeve is sunken into the cone; the hexagonal disk is arranged between the hemisphere and the cone, and a hemisphere end polyester membrane, a hexagonal disk polyester membrane and a cone end polyester membrane are respectively arranged in the meshed bracket. The patent is applied in the left atrial appendage occlusion surgery, however, the patent merely has one hexagonal disk, thus it may appear a slip-off phenomenon when occluding the left atrial appendage, and the hexagonal disk may cause harm on inner wall of the left atrial appendage in the meanwhile. Besides, the patent lacks structural diversity, and fails to abut its inner wall in accordance with the actual conditions of the patient's left atrial appendage, as a result, the effect thereof is not ideal.

### Summary of the Present Invention

The object of the present invention is to provide a left atrial appendage occluder, which is simple in structure, convenient for use, and enables to reduce the difficulty of the surgery, and may abut its inner wall in accordance with the actual condition of the left atrial appendage, so as to overcome the above defects in the prior art.

In order to solve the above technical problems, the present invention adopts the following technical solution: a left atrial appendage occluder, comprising a nitinol meshed bracket, wherein the nitinol meshed bracket has a tubular shape and is provided with a left disk and a right disk at two ends of the nitinol meshed bracket, respectively; the left disk and the right disk are connected by a mobile portion, the left disk and the right disk are provided with a resistance membrane in a transverse direction, respectively.

Preferably, the mobile portion is also provided with one or two layer of resistance membrane in a radial direction.

Preferably, the resistance membrane is made of polytetrafluoroethylene or polyethylene terephthalate.

Preferably, the left disk has a diameter smaller than that of the right disk.

Further, the mobile portion has a cone-shape, and the end of the mobile portion connecting to the left disk is thinner than the end connecting to the right disk.

Another object of the present invention is to provide a manufacture method of the above left atrial appendage occluder, with technical solutions as follows:
firstly, weaving the nitinol wires into a blank web of elongated tubular shape by using an automatic weaving machine or by hand weaving;
secondly, placing the woven blank web into a heat treatment resistance furnace, which is heated up to 250°∼350°, and is taken out after heat preservation;
thirdly, truncating the heat-treated blank web into 20∼100mm, and two ends of the truncating part are clamped and sealed by a stainless steel sleeve, or sealed by welding.
fourthly, performing shaping treatment on the sealed blank web by a shaping mould, wherein the shaping mould comprises a die, an inner core, an upper gland and an lower gland; placing the inner core of the shaping mould in the blank web from a mesh of the blank web, placing the blank web with the inner core in a cavity of the die of the shaping mould, then assembling the stainless steel sleeve or the welding head at the two ends of the blank web with the upper and lower glands of the shaping mould, finally pressing the upper and lower glands to fix on the cavity;
fifthly, placing the shaping mould with the blank web in a heat treatment resistance furnace, which is heated up to 500°∼550°, and is taken out after heat preservation, to form the nitinol meshed bracket;
sixthly, placing at least two layers of the resistance membrane of polytetrafluoroethylene or polyethylene terephthalate in the nitinol bracket, to finally form the left atrial appendage occluder.

Preferably, in the first step, the heating time is 10∼40min, and the heat preservationtime is 30∼60min.

Preferably, in the first step, the nitinol wires are woven onto a stainless steel round bar, which is further placed into the heat treatment resistance furnace after weaving, to perform heat treatment.

Preferably, in the fifth step, the heat preservation time is 10∼20min.

Preferably, in the sixth step, the resistance membrane of polytetrafluoroethylene or polyethylene terephthalate is fixed inside the nitinol bracket by stitching.

From the above, the left atrial appendage occluder and the manufacture method thereof in accordance with the present invention, has the following beneficial effects:
the present invention is applied in the surgical intervention, in which there is no need for establishing a vein-left atrial appendage track, but using a ultrasonic guidance instead; besides, there is no need for x-ray irradiation, thereby both the doctor and patient will not be suffered by the effect of the X-ray. In the meanwhile, during the surgery, through a small incision of the left posterior intercostal, one may directly view the left atrial appendage to directly use the present invention, which makes the surgical process simpler and quicker. The present invention also adopts double disks for occluding, while left disk of the occluder is in the left atrial appendage, which helps to effectively fix the occluder, and to prevent the occluder from slipping off. The concave-convex structure caused by different diameters of the two disks and the mobile portion, can effectively fix the occluder into the left atrial appendage. Moreover, once the occluder is placed in the left atrial appendage, the length of the occluder may be automatically adjusted according to the morphology of the left atrial appendage, which is convenient and reliable in use.

### Brief Description of the Drawings

Figure 1 is an initial state diagram of the manufacture of the present invention.
Figure 2 is a structural diagram of the shapingmould.
Figure 3 is a diagram showing the assembly of the present invention into the shaping mould.
Figure 4 is a first kind of structural diagram of the present invention.
Figure 5 is a second kind of structural diagram of the present invention.
Figure 6 is a third kind of structural diagram of the present invention.
Figure 7 is a fourth kind of structural diagram of the present invention.
Figure 8 is a diagram showing an embodiment during a surgery of the present invention.

Wherein:

| | |
|---|---|
| 1 nitinol meshed bracket | 11 left disk |
| 12 right disk | 13 mobile portion |
| 2 resistance membrane | 3 stainless steel sleeve |
| 4 left atrial appendage | 5 left atrium |
| 61 cavity | 62 inner core |
| 63 upper gland | 64 lower gland |

### Detailed Description of the Preferred Embodiments

The structure, the scale, the size and the like shown in the drawings attached in this specification are all simply used to match with the content exposed by the specification for the skilled in the art understanding and reading, but not used to limit qualifications when the invention may be implemented, thus any modification of structure, alteration of proportional relation, or modulation of size without technical essential meanings shall be fall into the covered scope by the disclosed technical solution of the invention, with no effect to the generated function and achieved objects of the present invention. Meanwhile, terms such as "up", "down", "left", "right" and the like cited in this specification are also simply for clearness of the description but not used to limit the scope implemented by the invention. The change or the adjustment of the relative relation should also be seen as the scope of the invention when there is no substantial alteration in the technical content.

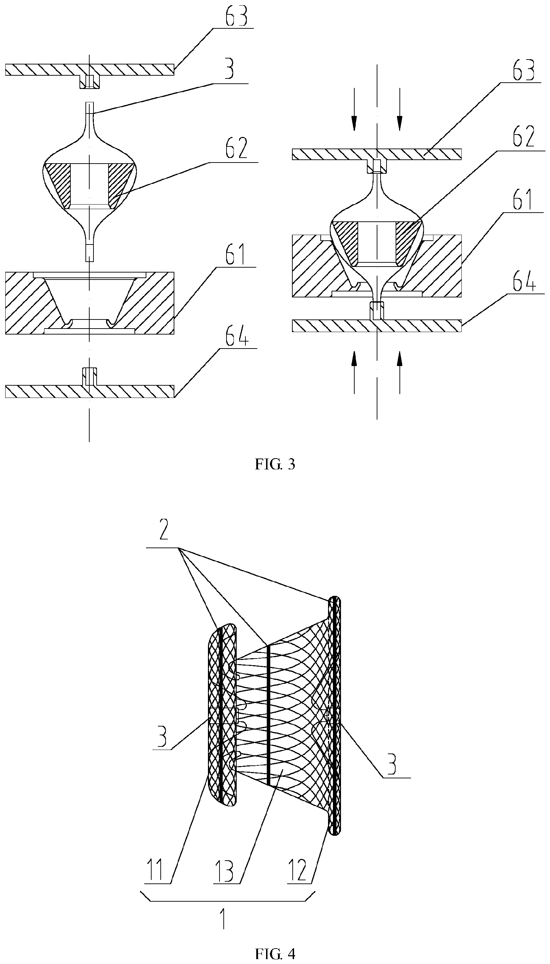

As shown in any of figures 4 to 7, a left atrial appendage occluder of the present invention, comprises a nitinol meshed bracket 1, wherein the nitinol meshed bracket 1 has a tubular shape, and is provided with a left disk and a right disk at two ends of the nitinol meshed bracket, respectively. The left disk 11 and the right disk 12 are connected by a mobile portion 13, and the mobile portion 13 may be stretched, compressed or twisted as required. The left disk 11 and the right disk 12 are provided with a resistance membrane 2 in a transverse direction, respectively. In the present embodiment, the resistance membrane 2 is made of polytetrafluoroethylene or polyethylene terephthalate.

In the present embodiment, in order to further block the flow of blood in practice, the mobile portion 13 is also provided with one or two layers of resistance membrane 2 in a transverse direction.

The present invention is applied in an occlusion surgery of a left atrial appendage. Since the left atrial appendage generally has a cone shape, in order to better abut the inner wall of the left atrial appendage to make the resistance membrane 2 ensure a sufficient flow resistance effect, the left disk 11 has a diameter smaller than that of the right disk 12. During the installation of the operation, the left disk 11 is placed at a thinner end of the left atrial appendage, and the right disk 12 is placed at a thicker end of the left atrial appendage. Further, In order to facilitate matching the inner cavity of the left atrial appendage, the mobile portion 13 has a cone-shape, the diameter of the left disk 11 is larger than the largest diameter of the left end of the mobile portion 13, the diameter of the right disk 12 is larger than the largest diameter of right end of the mobile portion 13, and the end of the mobile portion 13 connecting to the left disk 11 is thinner than the end connecting to the right disk 12. Since the present invention is occluded in the left atrial appendage, in order to prevent from slipping off, the diameters of both the left disk and right disk are larger than that of the inner cavity of the left atrial appendage.

The manufacture method of the left atrial appendage occluder comprises the following steps of:
firstly, weaving the nitinol wires into a blank web of elongated tubular shape by using an automatic weaving machine or by hand weaving. In order to facilitate weaving, the nitinol wires may be woven onto a stainless steel spindly round bar, and be fixed at the two ends of the round bar with hoops. During heat treatment in the next step, the woven blank web and the stainless steel round bar are placed into the heat treatment resistance furnace, by which deformation of the heat treated blank web can be avoided. In the present embodiment, the blank web refers to an embryonic form of the nitinol meshed bracket that is not completely shaped, but has an uniform elongated tubular shape.

During the heat treatment process, in order to prevent the nitinol wires from damage, e.g., fracture and crack of the wires, and in order to determine the time and temperature of the heat treatment process according to the size of the blank web, generally, the woven blank web is placed in a heat treatment resistance furnace for heating, with a temperature heated up to 250°∼350° within a heating time of 10∼40min. Wherein, it is preferred to heat the blank web to 250°, and after the preset processing time is achieved, the blank web stops heating for 30∼60min, then is taken out from the heat treatment resistance furnace, and is naturally cooled to room temperature in air.

After cooled to room temperature, the initial shaping of the blank web is finished. Then the heat treated blank web is truncated into 20∼100mm as required, and two ends of truncated blank web are clamped and sealed by a stainless steel sleeve 3, or sealed by welding.

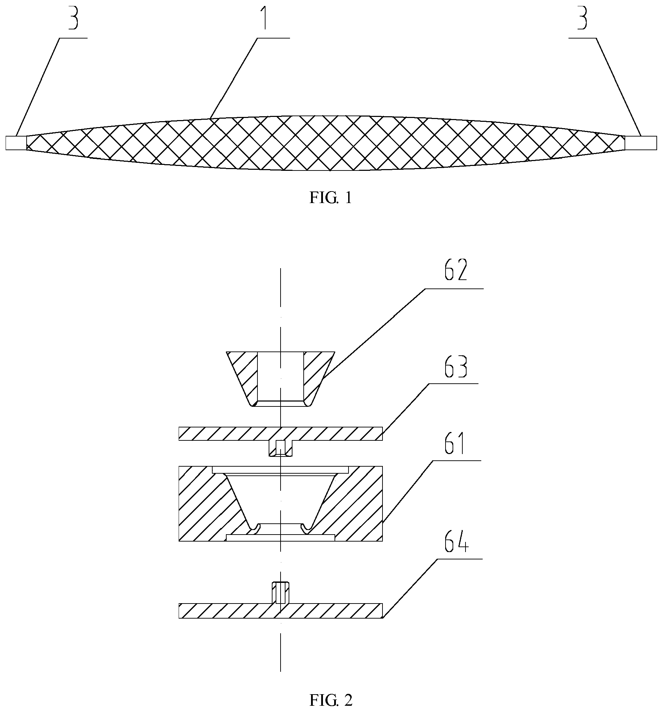

After sealing, the blank web needs to perform shaping treatment by using a shaping mould, wherein the shaping mould comprises a die 61, an inner core 62, an upper gland 63 and an lower gland 6. The die 61 has a cavity, the shape of which is adapted to the shape of the shaped left atrial appendage occluder (as shown in figures 4 to 6). In the present embodiment, by taking the two ends of the blank web as stainless steel sleeves 3 as an example, and in combination with figures 1 to 4, the operation steps thereof is that: meshes of the sealed blank web may be stretched to a certain extent, the inner core 62 of the shaping mould is placed into the blank web through a mesh; the blank web with the inner core 62 is placed in a cavity of the die 61, then the stainless steel sleeve 3 at the two ends of the blank web are coordinately assembled to corresponding fixing holes of the upper and lower glands 63, 64 of the shaping mould, finally, the upper and lower lower glands 63, 64 are pressed and fixed onto the die 61. During the pressing and fixing process, because the blank web itself has tension, and the external forces applied to the upper and lower glands 63, 64 may help to fill the blank web into the cavity of the die 61, and with the effect of the inner core 62 simultaneously, the blank web may finally form the left disk 11 and the right disk 12 in the cavity, as well as the twistable and stretchable mobile portion 13.

After that, the shaping mould, together with the blank web therein, is placed in the heat treatment resistance furnace to perform the final shape. The final shaped blank web should meet the following two requirements: one is to ensure the overall strength and thus ensure the support effect, from being slipped off; second is to ensure that the middle mobile portion 13 can twist or stretch to facility the cooperation with the inner cavity of the left atrial appendage. In order to achieve the above requirements, after a large amount of experiments, the inventors set the temperature of the heating treatment of the final shaping as 500°∼550°, and keep the temperature in the heat treatment resistance furnace for 10∼20min; after that, the blank web is taken out and quickly cooled in water to room temperature to complete the final shaping. After cooling to room temperature, the inner core is taken out from a mesh of the web, to form the nitinol meshed bracket 1.

Finally, the resistance membrane 2 of at least two layers of polytetrafluoroethylene (EPTFE) or polyethylene terephthalate (PET) is placed inside the shaped nitinol meshed bracket 1. The resistance membrane 2 is polymer structure. If the resistance membrane 2 is inserted into the mesh of the nitinol meshed bracket 1, the resistance membrane 2 will fully expand, and the expanded resistance membrane 2 is fixed in the nitinol meshed bracket by stitching sealing. Generally, the left disk 11 and the right disk 12 are provided with a layer of resistance membrane 2, respectively, and in order to further ensure the flow resisteance effect in the actual application, the mobile portion may be provided with at least one resistance membrane 2 as well. After the resistance membrane is assembled, a complete left atrial appendage occluder is finished.

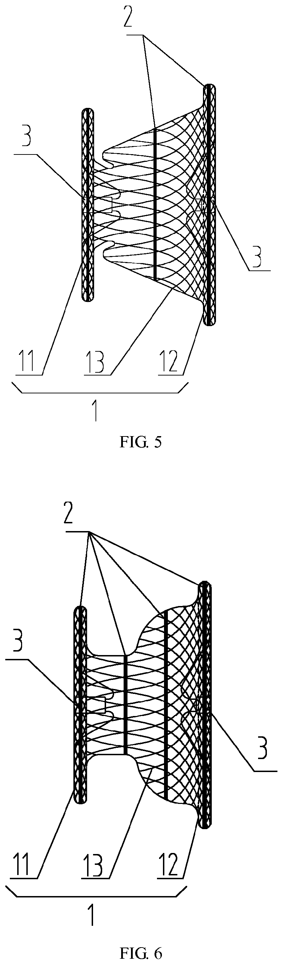

As shown in figures 4 to 6, there may be a plurality of structural forms of the mobile portion 13, such as, the tubular-like structure formed in figures 4 and 5, or the ladder-like structure formed in figure 6, or other forms such as corrugate-like structure, as long as it satisfies the twisting and stretching conditions. For example, left atrial appendage structure of different patients are generally different, the mobile portion 13 is designed as a variable waist shape to adpat to different patients better, the height of the occluder can be adjust by strecthing. In the meanwhile, the specific structure of the left disk 11 and the right disk 12 are determined according to actual situations. As an example, the left disk 11 in figure 4 is relative thicker, which may help to increase the contact area with the left atrial appendage, thereby the structure is more stable.

The occluder of the present invention is applied in the surgical intervention, in which there is no need for establishing a vein-left atrial appendage track, but cutting a small incision at the left posterior intercostal of the patient, through which the doctor can have a direct view of the left atrial appendage, then use a ultrasonic guidance. Thereby both the doctor and patient will not be suffered from the effect of the X-ray, which makes the surgical procedure simpler and quicker. In the present invention, the right disk 12 is outside of the left atrial appendage, which helps to effectively fix the occluder of the present invention, to prevent it from slipping off. The diameter of the mobile portion 13 between the left disk 11 and the right disk 12 is smaller than that of the left disk 11 and the right disk 12, i.e., to form a structure that is thick at both ends and is thin at the center, by which the occluder is effectively fixed to the left atrial appendage. Once the occluder is placed in the left atrial appendage, the length and shape of the mobile portion 13 may be automatically adjusted according to the morphology of the left atrial appendage, so that the mobile portion 13 can make the occluder automatically align the center of the left atrial appendage.

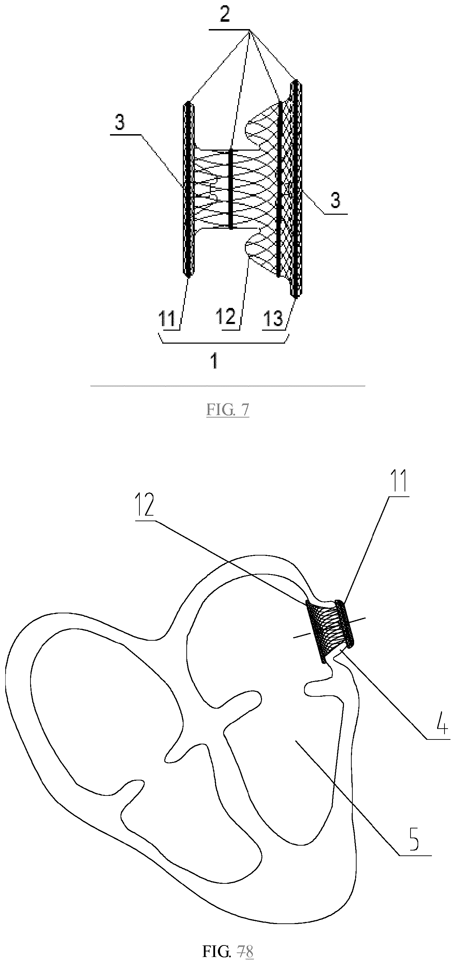

In combination with figures 4 and 7, which shows an embodiment of the present invention in operation, firstly the left posterior intercostal of the patient is cut for a small incision, then the left atrial appendage 4 is cut for an opening, a ultrasonic guidance is used to stretch the occluder of the present invention into a left atrium 5 through an inner cavity of the left atrial appendage 4, wherein the disk of the right disk 12 abuts the inner wall of the left atrium 5, the mobile portion 13 abuts the inner wall of the left atrial appendage 4, the left disk 11 is blocked at the opening of the left atrial appendage 4. The resistance membrane 2 in the right disk 12 may effectively prevent blood from entering the left atrial appendage 4, and the resistance membrane 2 in the middle mobile portion 13 may further block blood. Since the left disk 11 is blocked at the opening, the two ends of the the occluder sandwich the left atrial appendage, so that the occluder can neither slip into the left atrium 5, nor slip from the left atrial appendage 4. Besides, the resistance membrane 2 of the left disk 11 is to prevent the blood from outflowing the opening of the left atrial appendage 4.

From the above, the left atrial appendage occluder and the manufacture method thereof in accordance with the present invention, enable to solve the security problem of the occlusion surgery of the left atrial appendage, save operation time, and have good effect. Therefore, the present invention effectively overcomes a variety of disadvantages in the prior art and has high industrial utility value.

The abovementioned embodiments only illustratively describe the principle and efficacy of the present invention, rather than being used to limit the present invention. Any person skilled in the art may modify or amend the abovementioned embodiments without departing from the spirit and scope of the present invention. Thus, all equivalent modifications or amendments accomplished by persons having common knowledge in the technical field concerned without departing from the spirit and technical thoughts revealed by the present invention shall still be covered by the claims of the present invention.

## Claims

1. A left atrial appendage occluder, **characterized in that**: comprising a nitinol meshed bracket (1), wherein the nitinol meshed bracket (1) has a tubular shape, and is provided with a left disk and a right disk at two ends of the nitinol meshed bracket, respectively; the left disk (11) and the right disk (12) are connected by a mobile portion (13), the left disk (11) and the right disk (12) are provided with a resistance membrane (2) in a transverse direction, respectively.

2. The left atrial appendage occluder according to claim 1, **characterized in that**: the mobile portion (13) is also provided with one or two layer of resistance membrane (2) in a radial direction.

3. The left atrial appendage occluder according to claim 1, **characterized in that**: the resistance membrane (2) is made of polytetrafluoroethylene or polyethylene terephthalate.

4. The left atrial appendage occluder according to claim 1, **characterized in that**: the left disk (11) has a diameter smaller than that of the right disk (12).

5. The left atrial appendage occluder according to claim 4, **characterized in that**: the mobile portion (13) has a cone-shape, and the end of the mobile portion (13) connecting to the left disk (11) is thinner than the end connecting to the right disk (12).

6. A manufacture method of the left atrial appendage occluder according to claim 1, **characterized in that** 0, it comprises the following steps of:
firstly, weaving the nitinol wires into a blank web of elongated tubular shape by using an automatic weaving machine or by hand weaving;
secondly, placing the woven blank web in a heat treatment resistance furnace to heat up to 250°∼350°, and is taken out after heat preservation;
thirdly, truncating the heat-treated blank web into heat-treated, and two ends of the truncating part are clamped and sealed by a stainless steel sleeve, or sealed by welding;
fourthly, performing shaping treatment on the sealed blank web by a shaping mould, wherein the shaping mould comprises a die (61), an inner core (62), an upper gland and an lower gland (63, 64);
placing the inner core (62) of the shaping mould in the blank web from a mesh of the blank web, placing the blank web with the inner core (62) in a cavity of the die (61) of the shaping mould, then assembling the stainless steel sleeve or the welding head at the two ends of the blank web with the upper and lower glands of the shaping mould, finally pressing the upper and lower glands to fix on the cavity;
fifthly, placing the shaping mould with the blank web in a heat treatment resistance furnace, which is heated up to 500°∼550°, and is taken out after heat preservation incubation to form the nitinol meshed bracket (1);
sixthly, placing at least two layers of the resistance membrane (2) of polytetrafluoroethylene or polyethylene terephthalate in the nitinol bracket (1), to finally form the left atrial appendage occluder.

7. The manufacture method of the left atrial appendage occluder according to claim 6, **characterized in that**: in the first step, the heating time is 10∼40min, and the heat preservation time is 30∼60min.

8. The manufacture method of the left atrial appendage occluder according to claim 6, **characterized in that**: in the first step, the nitinol wires are woven onto a stainless steel round bar, which is further placed into the heat treatment resistance furnace after weaving, to perform heat treatment.

9. The manufacture method of the left atrial appendage occluder according to claim 6, **characterized in that**: in the fifth step, the heat preservation time is 10∼20min.

10. The manufacture method of the left atrial appendage occluder according to claim 6, **characterized in that**: in the sixth step, the resistance membrane (2) of polytetrafluoroethylene or polyethylene terephthalate is fixed in the the nitinol bracket (1) by stitching.
